# EUROPÄISCHE PATENTSCHRIFT

(11) **EP 2 725 976 B1**
(45) Veröffentlichungstag und Bekanntmachung des Hinweises auf die Patenterteilung: **21.04.2021**
(21) Anmeldenummer: 12737187.0
(22) Anmeldetag: 29.06.2012
(51) Int. Cl.: A61B 5/11, A61B 5/00

(54) **MESSVORRICHTUNG ZUM ERFASSEN VON LAGEÄNDERUNGEN VON PERSONEN IN BETTEN**
MEASURING DEVICE FOR DETECTING POSITIONAL CHANGES OF PERSONS IN BEDS
DISPOSITIF DE MESURE POUR DÉTECTER DES MODIFICATIONS DE POSITION DE PERSONNES SUR DES LITS

(30) Priorität: 01.07.2011 CH 11122011
(43) Veröffentlichungstag der Anmeldung: 07.05.2014
(73) Patentinhaber: Compliant Concept AG, 8600 Dübendorf (CH)
(72) Erfinder: MAGGI, Andrin, CH-8037 Zürich (CH); SAUTER, Michael, CH-8413 Neftenbach (CH)
(74) Vertreter: Gachnang, Hans Rudolf
(86) Internationale Anmeldenummer: PCT/CH2012/000146
(87) Internationale Veröffentlichungsnummer: WO 2013/003963

(56) Entgegenhaltungen:
- EP-A1- 0 671 145
- WO-A1-2009/127405
- US-A- 5 780 781
- US-A1- 2007 174 964
- US-A1- 2008 275 349
- US-A1- 2011 112 442

## Beschreibung

Gegenstand der Erfindung ist eine Messvorrichtung zum Erfassen von Lageänderungen von Personen in Betten gemäss dem Oberbegriff des Patentanspruchs 1, ein Bett mit einer Messvorrichtung gemäss dem Oberbegriff des Patentanspruchs 10 und ein Verfahren zum Erfassen von Messgrössen bei einem Bett gemäss dem Oberbegriff des Patentanspruchs 11.

Liegt eine Person längere Zeit ohne sich zu bewegen, so können an den belasteten Druckstellen des Körpers Druckgeschwüre entstehen. Dabei werden die Haut und das darunter liegende Gewebe lokal geschädigt. Solche Druckgeschwüre werden auch Dekubitus, Dekubitalgeschwüre oder Wundliegegeschwüre genannt.

Bei gesunden Personen ist die Gefahr von Dekubitus gering, da sie ihre Lage selbst im Schlaf oft ändern. Bei pflegebedürftigen Personen hingegen kann die Fähigkeit zur selbständigen Lageänderung beeinträchtigt oder gestört sein. Sie müssen deshalb in der Regel von Pflegepersonal überwacht und regelmässig umgelagert werden. Die Überwachung und Umlagerung gefährdeter Personen durch betreuende Pflegepersonen ist aufwändig und teuer. Das Dekubitus-Gefahrenpotential wird oft nicht richtig eingeschätzt. Bei überlasteten und/oder ungenügend geschulten Pflegepersonen besteht somit die Gefahr, dass eine pflegebedürftige Person einen Dekubitus erleidet.

Dies wiederum führt zu Folgekosten bei der Pflege dieser Personen, welche bei geeigneten Vorsorgemassnahmen vermeidbar wären.

Die deutsche Patentanmeldung DE4240782A1 offenbart ein Verfahren und eine Vorrichtung zur Dekubitusprophylaxe, wobei Druckwerte von Körperpartien eines Patienten gemessen werden. Dabei liegt die Person auf einer weichelastischen Matte mit einer Vielzahl von Drucksensoren, die in einer Gitterstruktur angeordnet sind. Die Matte ist gross genug, dass mindestens der Körperbereich von den Schultern bis zum Gesäss auf dem Messbereich aufliegt. Die Matte ist glatt, gut an die Körperform anpassbar und aus einem waschbaren und/oder sterilisier- bzw. desinfizierbaren Kunststoff gefertigt.

Die Sensoren werden über Analog/Digital-Wandler mit einem Rechner bzw. Notebook zur Auswertung der Messdaten verbunden. Dieser wertet die Druckdaten der Sensoren aus und erzeugt einen optischen oder akustischen Alarm, wenn die Druckbelastung der überwachten Person zu gross ist und die Person umgebettet bzw. umgelagert werden muss.

Die Vielzahl von Sensoren ermöglicht innerhalb eines relativ grossen Messbereichs eine differenzierte Erfassung der Druckverteilung. Die Auswertung der Sensorsignale ist aber relativ komplex. Für eine wirksame Dekubitus-Prophylaxe ist eine genaue Kenntnis lokaler Druckstellen und der Druckverteilung über einen Grossteil der Liegezone nicht zwingend erforderlich. Grundsätzlich würde es genügen, Lageänderungen einer Person zu erfassen, die eine ausreichende Entlastung gefährdeter Druckstellen bewirken. Da die zu überwachende Person im Wesentlichen direkt auf der Matte aufliegen muss, kann die Wirksamkeit gegebenenfalls vorhandener anderer Mittel, die zur situationsgerechten Lagerung und/oder Überwachung der Person unterhalb der Matte angeordnet sind - also beispielsweise in oder angrenzend an eine Matratze - beeinträchtigt werden. Zudem muss die Matte hohen hygienischen Standards genügen. Die Reinigung und Desinfektion verursacht zusätzlichen Wartungsaufwand. Im Weiteren kann sich die Vielzahl von Messleitungen zwischen den Sensoren und dem A/D-Wandler beim Betrieb und bei der Reinigung der Matte störend auswirken.

EP0671145A1 betrifft eine Sensoreinrichtung, welche dazu ausgebildet ist, die Anwesenheit eine Person in einem Bett zu überwachen. In einer ersten Ausführungsform umfasst die Sensoreinrichtung zwei rechteckige Stahlplatten, die entlang ihrer kürzeren Seiten durch Abstandhalter beabstandet zueinander gehalten sind. Zentral zwischen den Abstandhaltern ist ein Mikroschalter oder ein Punktsensor mit zwei zusammenwirkenden Teilen angeordnet. Wenn die Durchbiegung der oberen Platte einen Minimalwert unterschreitet, kann ein Alarm ausgelöst werden. Bei alternativen Ausführungsformen wird lokal die Durchbiegung oder eine Auflagekraft einer Platte erfasst, die als Liegefläche eines Bettes verwendet wird.

US2011/0112442A1 offenbart eine Vorrichtung mit einem Bewegungssensor zum Überwachen eines Patienten in einem Bett. Der Bewegungssensor kann insbesondere ein piezoelektrischer Sensor oder eine gitterartige Anordnung mehrerer piezoelektrischer Sensoren sein. Mit solchen Sensoren können durch Vergleich mit vorgegebenen Mustern Körperfunktionen wie z.B. der Herzschlag oder die Atmung überwacht werden. Solche Muster können auch für unterschiedliche Bewegungen eines Patienten vorgegeben sein. Zusätzlich kann die Vorrichtung mehrere Gewichtssensoren umfassen, mit denen zur Verbesserung der Genauigkeit das Gewicht oder die Schwerpunktlage eines auf dem Bett liegenden Patienten bestimmt werden kann.

Aus der US2007/0174964A1 ist ein Spitalbett bekannt, das eine Messvorrichtung zum Erfassen und Überwachen der auf einer Liegefläche aufliegenden Last umfasst. Die Liegefläche umfasst einen Kopfabschnitt, einen Sitzabschnitt und einen Fussabschnitt, die schwenk- und verschiebbar an einem Lastrahmen gelagert und von diesem Lastrahmen getragen sind. Der Lastrahmen ist als Ganzes im Bereich seiner vier Ecken über je eine Wägezelle auf einem Zwischenrahmen abgestützt. Die Wägezellen ermöglichen die Erfassung von Gewichtsschwankungen und der Schwerpunktlage eines Patienten, der auf einer von der Liegefläche getragenen Matratze liegt.

US5780781A offenbart eine Messvorrichtung mit mehreren Modulen, die durch flexible Bänder miteinander verbunden sind. Die Form und Grösse dieser Module sind abgestimmt auf bewegliche Abschnitte der Auflagefläche eines bestimmten Bettes. Die verbundenen Module bilden gemeinsam eine Liegefläche zum Auflegen einer Matratze. Jedes Modul umfasst zwei Kunststoffschalen, die durch Rippen verstärkt sein können. Bei jedem Modul sind an zwei sich gegenüberliegenden Endbereichen Gewichtssensoren zwischen den beiden Schalen angeordnet.

Eine Aufgabe der vorliegenden Erfindung ist es deshalb, eine Messvorrichtung für Betten zu schaffen, die es ermöglicht, Lageänderungen von Personen auf diesen Betten auf einfache Art zu erfassen und auszuwerten. Der Begriff "Bett" umfasst beliebige Liegevorrichtungen mit einer Matratze oder einer entsprechenden anderen Liegeunterlage. Dazu gehören insbesondere Pflegebetten und Betten, bei denen eine Matratze auf einer Liegefläche wie z.B. einem Lattenrost aufliegt, und bei denen diese Liegefläche von einem Bettgestell getragen ist.

Eine weitere Aufgabe der Erfindung besteht darin, die Messvorrichtung so auszubilden, dass sie mobil ist und auch nachträglich bei bestehenden Betten angewendet werden kann, und dass der Aufwand für deren Installation und Wartung möglichst gering ist.

Eine weitere Aufgabe der Erfindung besteht darin, ein Bett mit einer Messvorrichtung zur Dekubitus-Prophylaxe zu schaffen, wobei diese Messvorrichtung andere Funktionen des Bettes möglichst nicht beeinträchtigt.

Eine weitere Aufgabe der Erfindung besteht darin, ein Verfahren zur Verarbeitung von Messgrössen der Messvorrichtung zu schaffen, mit dem zur Verhinderung von Dekubitus erforderliche Umlagerungen einer auf dem Bett liegenden Person erkannt und signalisiert werden können.

Diese Aufgaben werden gelöst durch eine Messvorrichtung gemäss den Merkmalen des Patentanspruchs 1, durch ein Bett gemäss den Merkmalen des Patentanspruchs 10 und durch ein Verfahren zur Verarbeitung von Messgrössen gemäss den Merkmalen des Patentanspruchs 11.

Die Erfindung beruht auf der Erkenntnis, dass die Dekubitus-Gefahr bei einer in einem Bett liegenden Person massgeblich aufgrund von regelmässigen Lageänderungen insbesondere im Beckenbereich dieser Person bzw. aufgrund von Änderungen der Lage des Schwerpunktes der im Gesäss- oder Beckenbereich auf die Matratze wirkenden Gewichtskraft beurteilt werden kann. Lageänderungen des Beckenbereichs gehen einher mit Bewegungen oder Änderungen des Auflagedrucks bei anderen Körperpartien, die ebenfalls gefährdet sind. Wenn die Person die Lage ihres Beckens bzw. die Gewichtsverteilung im Beckenbereich autonom innerhalb einer vorgegebenen Zeitspanne mehrmals ändert, ist die Dekubitus-Gefahr gering. Zur rechtzeitigen Erkennung des Risikos für ein Druckgeschwür genügt es deshalb, Bewegungen bzw. Lageänderungen im Beckenbereich einer Person zu überwachen. Dies wird erreicht, indem in einem mittleren Bereich der Liegefläche eines Bettes die Lage des Schwerpunktes der von diesem mittleren Bereich getragenen Last quer zur Längsrichtung der Liegefläche erfasst wird. Der Beckenbereich der zu überwachenden Person wird in diesem mittleren Bereich der Liegefläche abgestützt. Bewegt sich die Person, so ändert sich die Verteilung der Gewichtslast quer zum Bett und damit auch die Lage des Schwerpunkts in diesem mittleren Bereich. Zum Erfassen der Schwerpunktlage quer zum Bett ist in einem mittleren Bereich der Liegefläche zwischen deren Kopfbereich und Fussbereich eine Brückenkonstruktion angeordnet, welche die Matratze im Beckenbereich einer darauf liegenden Person stützt. Die Brückenkonstruktion - fortan auch kurz Brücke genannt - umfasst bei einer ersten Ausführungsform eine biegesteife Querlatte bzw. eine längliche Platte, deren Länge zumindest näherungsweise der Breite des Bettes entspricht, bei dem sie verwendet werden soll. Mindestens im Bereich ihrer beiden Enden umfasst die Platte Stützstellen bzw. Stützbereiche die zum Stützen, Tragen, Lagern oder Halten am Bettgestell bzw. allgemein an einer Tragvorrichtung geeignet sind. Je nach Ausführungsform der Brücke und des Bettes kann die Abstützung beispielsweise direkt an seitlich der Liegefläche ausgebildeten Längsholmen eines Bettgestells erfolgen. Die Abstützung der Platte am Gestell kann auch indirekt über Verbindungsmittel erfolgen. Solche Verbindungsmittel können Elemente des Bettgestells und/oder Elemente der Messvorrichtung umfassen. Insbesondere können Querstreben des Bettgestells oder Latten eines Lattenrostes als Verbindungsmittel genutzt werden.

Bei einer besonders vorteilhaften Ausgestaltung der Erfindung umfasst die Brücke in geringem Abstand unterhalb der biegesteifen Platte eine weitere Platte mit höherer Biegeelastizität. Die obere, biegesteife Platte ist zumindest im Bereich der Stützstellen an den beiden kürzeren Enden mittels Stützkörpern bzw. Abstandhaltern auf der unteren Auflageplatte abgestützt. Sensoren zum Erfassen der lokal abgestützten Gewichtskraft können im Bereich der Stützstellen an der Platte selbst, an den Stützkörpern oder an der darunter angeordneten Auflageplatte bzw. allgemein an Verbindungsmitteln ausgebildet sein. Aufgrund ihrer Biegeelastizität kann die Auflageplatte bei unterschiedlichen Tragkonstruktionen bzw. Bettgestellen abgestützt werden. Die Stützkörper liegen selbst dann auf der unteren Platte auf, wenn sich unmittelbar darunter eine Lücke oder Vertiefung im Bettgestell befindet. Das auf der Brücke lastende Gewicht kann bei jedem Bett zuverlässig auf die beiden Längsholmen des Bettgestells übertragen werden, wobei die Sensoren Informationen über die Verteilung der Gewichtslast auf die beiden Längsholmen liefern.

Vorzugsweise umfasst die Brücke eine waschbare, abnehmbare Hülle aus Kunststoff, in der die beiden Platten eingebettet sind. Die Steuerung zur Auswertung der Sensorsignale kann teilweise oder vollständig z.B. zwischen den beiden Platten innerhalb der Hülle angeordnet sein.

Vorzugsweise wird zumindest ein Teil der Steuerung mit Analog-Digital-Wandlern zur Digitalisierung der Sensorsingale im Bereich der Brücke, beispielsweise zwischen den beiden Platten angeordnet. Dadurch kann der Einfluss von Störsignalen minimiert werden. Die Elektronik im Inneren der Hülle kann über ein nach aussen geführtes Anschlusskabel mit einem ausserhalb der Brücke angeordneten Teil der Steuerung verbunden werden.

Alternativ kann auch eine Funkverbindung für die Kommunikation zwischen einem Steuerungsteil innerhalb der Hülle und einem externen Steuerungsteil verwendet werden.

Wenn im Bereich der Brücke zusätzlich ein Akku angeordnet ist, der z.B. mittels einer induktiven Ladevorrichtung kontaktlos aufladbar ist, kann die Hülle optional hermetisch geschlossen sein. Aus den Messgrössen der Sensoren kann die Steuerung Änderungen der auf der Platte bzw. allgemein auf der Brücke bzw. Querverbindung lastenden Gewichtsverteilung herleiten.

Bei einer bevorzugten Ausführungsform umfasst die Brücke einen oder mehrere resistive Kraft- oder Drucksensoren, insbesondere Dehnmessstreifen, auch kurz DMS genannt, die abhängig von der jeweiligen Druck- oder Zugbelastung ihren elektrischen Widerstand ändern. Alternativ können auch nach anderen physikalischen Prinzipien arbeitende Sensoren verwendet werden, deren Messgrössen sich abhängig von der auf die Brücke einwirkenden Lastverteilung ändern, beispielsweise optische, kapazitive oder induktive Sensoren, welche eine Verformung der Brücke in Abhängigkeit der Gewichtslastverteilung erfassen können.

Grundsätzlich können auch piezoelektrische Kraftsensoren verwendet werden. Diese sind in der Regel aber nur zur Erfassung dynamischer Vorgänge geeignet. Für die Erfassung einer statischen Gewichtslastverteilung oder der Schwerpunktlage quer zum Bett sind aktive piezoelektrische Wandler aufgrund ihres Drifts eher ungeeignet. Die Erfassung von Änderungen der Gewichtslastverteilung ist aber möglich.

Bei einer Ausführungsform der Brücke mit nur einem Gewichtssensor ist dieser vorzugsweise so angeordnet, dass er möglichst nur die Auflagekraft des Querelementes auf der einen Seite des Gestells erfasst. Abhängig von der Schwerpunktlage im Beckenbereich der aufliegenden Person ändert sich bei der Brücke die Gewichtslastverteilung zwischen den beiden seitlichen Stützbereichen am Gestell.

Die vom Sensor erfasste Messgrösse ändert sich somit, wenn die Schwerpunktlage im Beckenbereich der zu überwachenden Person sich ändert. Zur Erfassung und Auswertung der Messgrösse ist der Sensor mit einer Steuerung verbunden. Sowohl die obere biegesteife Platte bzw. die biegesteifen Plattenabschnitte als auch die biegeelastische Auflageplatte sind aus Metall, vorzugsweise aus rostfreiem Stahl gefertigt. Zur Erhöhung der Biegesteifigkeit umfassen die obere Platte bzw. die oberen Plattenabschnitte abgekantete bzw. um 90° umgebogene Längskanten und/oder in Längsrichtung verlaufende Verstärkungsrippen. Letztere können beispielsweise durch Tiefziehen an der Platte selbst ausgeformt sein. Alternativ oder zusätzlich können Verstärkungselemente auch in Gestalt von länglichen Profilen aus Kunststoff oder anderen Materialien auf die Platte aufgeklebt oder in geeigneter anderer Weise mit der Platte verbunden werden.

Bei alternativen Ausführungsformen der Brücke können zwischen den Stützbereichen bei den äusseren Enden der Platte zusätzlich weitere Stützbereiche ausgebildet sein, welche direkt oder indirekt am Gestell bzw. der Tragvorrichtung gelagert sind, z.B. abgestützt mittels Stützelementen auf einer darunter angeordneten biegeelastischen Auflageplatte, auf einer Bettlatte oder einer Querverbindung des Bettgestells oder der Liegefläche.

Bei einer weiteren vorteilhaften Ausgestaltung umfasst die die obere Platte der Brücke mehrere - beispielsweise zwei oder drei - zusammengefügte biegesteife Abschnitte. Die Verbindungen benachbarter Plattenabschnitte können z.B. gelenkig oder allgemein flexibel durch Bänder, durch Nieten, Schweissen, Kleben oder in sonstiger Weise erfolgen. Zusätzliche Sensoren können im Bereich der Verbindungsstellen benachbarter Plattenabschnitte angeordnet sein. Solche Brücken können auch bei Betten mit gewölbten Liegeflächen, beispielsweise auf eine Bettlatte oder auf zwei benachbarte Bettlatten eines Lattenrosts aufgelegt werden und passen sich an die Form dieser Liegeflächen an. Alternativ könnten auch bei einer einstückigen Platte zwischen Abschnitten mit grosser Biegesteifigkeit flexible Zonen ausgebildet werden, indem die Platte dort beispielsweise eine geringere Stärke und/oder keine die Biegesteifigkeit erhöhenden Verstärkungselemente aufweist.

Optional können die Platten oder Plattenabschnitte bei den Stütz- bzw. Auflagebereichen Einschnitte bzw. Ausnehmungen umfassen, welche sprungbrettartige, geringfügig elastisch verformbare Zungen freilegen, die nur über schmale Verbindungsstege mit den angrenzenden Plattenbereichen verbunden sind. Im Bereich dieser Verbindungsstege sind Sensoren in Gestalt von Dehnmessstreifen angeordnet.

Grundsätzlich können jeweils ein oder mehrere Stützbereiche der Metallplatte an einem gemeinsamen Verbindungsmittel abgestützt sein, welches selbst wiederum am Gestell gelagert ist. Das Verbindungsmittel dient zur Kraftübertragung zwischen der Brücke oder der oberen Platte und dem Gestell. Bei alternativen Ausführungsformen können ein oder mehrere Sensoren auch an einem Verbindungsmittel selbst oder zwischen dem Verbindungsmittel und der Brücke bzw. der Platte oder zwischen dem Verbindungsmittel und dem Gestell ausgebildet sein.

Die Messvorrichtung mit der erfindungsgemäss ausgebildeten Brücke ist portabel und kann so ausgebildet sein, dass sie sowohl bei unterschiedlichen Betten als auch bei auf dem Boden aufliegenden Matratzen anwendbar ist, und dass bei der Installation keinerlei Werkzeug erforderlich ist. Dank einer geringen Bauhöhe von vorzugsweise weniger als 20 bis 25mm kann die Brücke bei unterschiedlichen Betten ohne zu stören zwischen Matratze und Liegefläche des Bettgestells platziert werden. Die geringe Breite der Brücke ermöglicht den Einsatz bei unterschiedlichen Betten. Die Messvorrichtung kann ohne Beeinträchtigung der Funktion eine Hülle aus einem feuchtigkeitsabweisenden Kunststoff als Spritzwasserschutz umfassen.

Bei einer weiteren alternativen Ausgestaltung der Erfindung kann die Brücke einen oder mehrere biegeschlaffe Abschnitte umfassen, wobei diese z.B. analog zur Bespannung einer Gartenliege in einem Rahmen zwischen den Längsholmen gespannt sind und auf Zug belastbare Sensoren umfassen. Bei einer solchen Ausführungsform sind die Sensoren vorzugsweise auf Zug belastbare Dehnmessstreifen im Randbereich der Bespannung bzw. der Brücke. Der Begriff "Stützbereiche" ist demnach weit auszulegen. Er umfasst nicht nur die Zonen oder Bereiche einer biegesteifen Brücke, die direkt oder indirekt am Gestell abgestützt werden, sondern allgemein jene Zonen oder Bereiche einer Brücke oder der Verbindung zwischen der Brücke und dem Gestell, an denen Kräfte oder Drehmomente zum Tragen der Brücke an das Gestell übertragen werden.

Die Steuerung der Messvorrichtung umfasst vorzugsweise im Bereich der Brücke eine Auswerteelektronik mit einer Analog-Digital-Wandlervorrichtung zum Erfassen und Digitalisieren der Sensorsignale und in einem separaten externen Gehäuse einen Steuerungsteil zum Auswerten der Sensorsignale sowie zum Ausgeben von Alarmsignalen. Alternativ können die Sensorsignale z.B. auch direkt vom Steuerungsteil bei der Brücke weiter verarbeitet und ausgewertet werden, beispielsweise mittels eines dort angeordneten Prozessors. Vorzugsweise ist die Steuerung so ausgebildet, dass sie den zeitlichen Verlauf der Sensorsignale speichern kann. Alternativ können auch aus der Verarbeitung der Messgrösse hergeleitete Informationen gespeichert werden. So kann die Steuerung beispielsweise Verarbeitungsvorschriften umfassen, welche aus der Messgrösse Bewegungen der Person herausfiltern, die zur Verhinderung von Dekubitus bei dieser Person erforderlich sind. Vorzugsweise werden nur Bewegungen mit einer vorgebbaren minimalen Stärke als relevante Bewegungen erfasst. Dabei können zusätzliche Kriterien wie das zeitliche Verhalten oder Beschleunigungen berücksichtigt werden.

Entspricht die Häufigkeit und Intensität der Bewegungen nicht gewissen in der Steuerung vorgegebenen Vergleichskriterien, so gibt die Steuerung ein Alarmsignal aus. Vorzugsweise können der Steuerung für jede zu überwachende Person in Abhängigkeit des jeweiligen Gesundheitszustandes individuelle Referenintervalle vorgegeben werden. Übersteigt die Dauer zwischen zwei aufeinanderfolgenden relevanten Bewegungen bei einer zu überwachenden Person die vorgegebene Dauer eines solchen Referenzintervalls, wird ein Alarmsignal ausgegeben. Vorzugsweise umfasst das externe Steuergerät der Steuerung eine Anzeige mit einer grünen, einer gelben und einer roten Anzeigeleuchte entsprechend einer Verkehrsampel. Übersteigt die Dauer ohne erfasste relevante Bewegungen den ersten Referenzwert, so wechselt die Anzeige von grün auf gelb. Übersteigt die Dauer ohne erfasste relevante Bewegungen den zweiten Referenzwert, so wechselt die Anzeige von gelb auf rot. Zusätzlich oder alternativ können auch akustische Alarmsignale ausgegeben und/oder über entsprechende Kommunikationsverbindungen Alarmeinrichtungen bei einem Patientenzimmer oder bei einer zentralen Überwachungsstation aktiviert werden.

Die Steuerung kann zusätzlich auch zum Erfassen von Referenzgrössen ausgebildet sein, welche bei der Verarbeitung der Messgrössen mitbenutzt werden können. So können beispielsweise von dem oder den Sensoren erfasste Messgrössen gespeichert werden, wenn die Person in einer definierten Lage auf dem Bett liegt, insbesondere dann, wenn die schwerpunktmässige Belastung der Brücke etwa in deren Mitte der Brücke erfolgt. Alternativ oder zusätzlich können der Steuerung auch das Körpergewicht der zu überwachenden Person oder andere Parameter vorgegeben werden. Zusammen mit diesen Parametern und der Messgrösse des Sensors kann die Steuerung nicht nur die zur Dekubitusprophylaxe erforderlichen Lageänderungen, sondern auch die Position der Person in Querrichtung des Bettes überwachen. Alternativ oder zusätzlich kann die Messgrösse beispielsweise auch in unterschiedlicher Weise gefiltert bzw. in unterschiedlicher Weise weiterverarbeitet werden, um daraus Informationen über Körperfunktionen wie etwa die Atmung oder den Puls zu gewinnen.

Vorzugsweise umfasst die Messvorrichtung zwei oder mehrere Sensoren. Mindestens je einer dieser Sensoren ist im Bereich der äussersten Stützbereiche der Brücke so angeordnet, dass er die Auflagekraft der Brücke auf dem Gestell in diesem Bereich erfassen kann.

Zwischen den beiden äussersten Stützbereichen kann die Brücke weitere Sensoren umfassen.

Anhand einiger Figuren wird die Erfindung im Folgenden näher beschrieben. Dabei zeigen
- Figur 1: eine erste Anordnung einer Brücke bei der Liegefläche eines Bettes,
- Figur 2: eine Detailansicht der Anordnung aus Figur 1 im Bereich von Übertragungselementen zum Abstützen einer Brücke auf einem Trägerelement mit Sensoren,
- Figur 3: einen Querschnitt der Anordnung aus Figur 1 im Bereich eines Übertragungselementes,
- Figur 4: eine zweite Anordnung einer Brücke bei der Liegefläche eines Bettes,
- Figur 5: die Brücke aus Figur 4 in einer Seitenansicht und in Aufsicht,
- Figur 6: eine schematische Darstellung der Abstützung und der Messgrössenerfassung bei Brücken mit einem oder mehreren Sensoren,
- Figur 7: eine Anordnung von mehreren Brücken bei der Liegefläche eines Bettes,
- Figur 8: eine weitere Brücke in perspektivischer Ansicht,
- Figur 9: eine schematisch dargestellte Seitenansicht der Anordnung aus Figur 8.

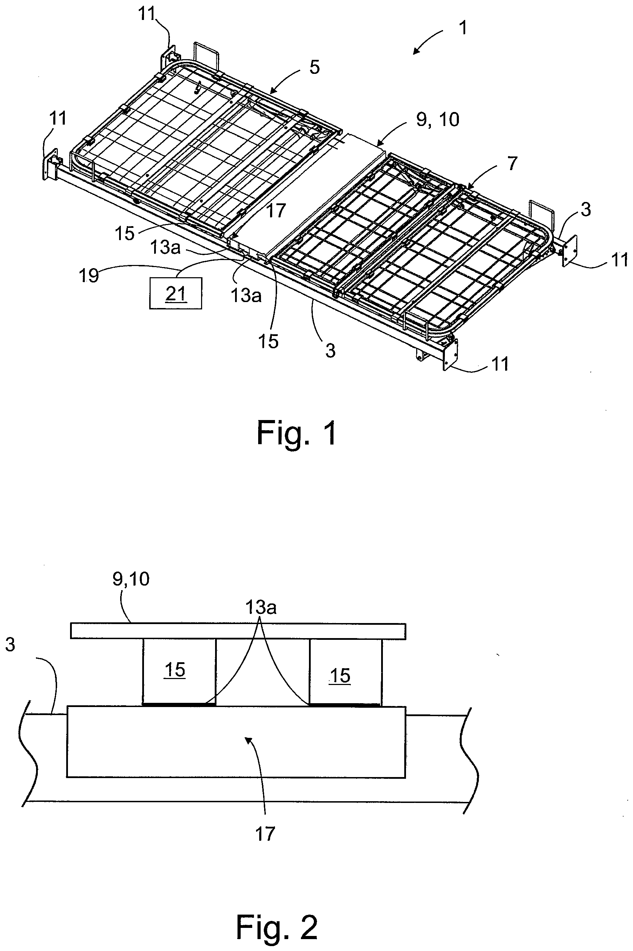

Figur 1 zeigt eine Liegefläche 1, wie sie z.B. bei Betten in Spitälern und Pflegeheimen zum Auflegen einer Matratze benutzt wird. Die Liegefläche 1 umfasst zwei parallele Längsholme 3, zwischen denen ein Rückenteil 5 und ein Bein- und Fussteil, fortan kurz Fussteil 7 genannt, schwenkbar gelagert sind. An den Enden der Längsholmen 3 sind Befestigungsplatten 11 zum Befestigen an einem Bettgestell (nicht dargestellt) - fortan auch kurz Gestell genannt - ausgebildet. Die Längsholme 3 können bei alternativen Ausgestaltungen der Betten auch als Bestandteil des Gestells ausgebildet sein, welches die Liegefläche 1 trägt. Zwischen dem Rückenteil 5 und dem Fussteil 7 umfasst die Liegefläche 1 bei herkömmlichen Betten ein Mittelteil 9, beispielsweise eine starre Blechkonstruktion, welche in der Regel fest mit den beiden Längsholmen 3 verbunden und relativ dazu nicht beweglich ist (nicht dargestellt). Das Mittelteil 9 erstreckt sich quer zum Bett etwa im Bereich des Beckens einer auf dem Bett liegenden Person.

Erfindungsgemäss wird dieses Mittelteil 9 als Brücke 10 ausgebildet oder durch eine solche Brücke 10 ergänzt oder ersetzt. Diese Brücke 10 ist Bestandteil einer Messvorrichtung mit mindestens einem Sensor 13, dessen Messgrösse in Abhängigkeit der auf die Brücke 10 einwirkenden Lastverteilung veränderbar ist. Die Brücke 10 ist als Kraftübertragungsmittel ausgebildet. Sie trägt als Teil der Liegefläche 1 das auf ihr lastende Gewicht und wird selbst wiederum von den Längsholmen 3 bzw. dem Bettgestell getragen.

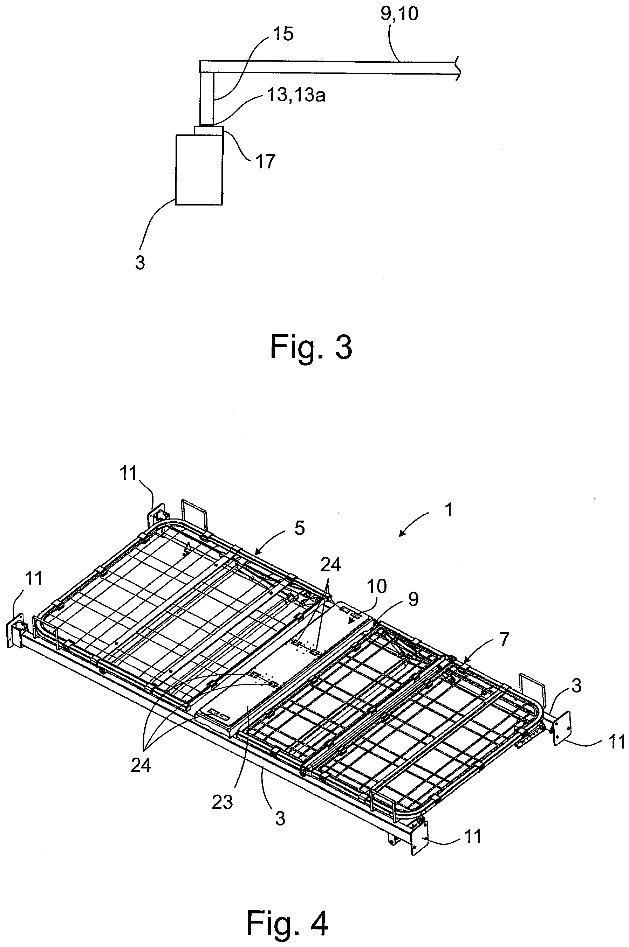

Bei einer ersten Ausführungsform gemäss Figur 1 umfasst die Brücke 10 eine starre, entlang ihrer Längskanten abgewinkelte Metallplatte. Im Bereich jeder der beiden Schmalkanten ist diese Metallplatte von Verbindungsmitteln in Gestalt von je zwei beispielsweise quaderförmigen Übertragungselementen 15 auf je einer Wiegefläche eines Kraftsensors 13a abgestützt. Die Wiegeflächen können beispielsweise als federnde Zungen eines L-förmig abgewinkelten, am jeweiligen Längsholm 3 befestigten Trägerelementes 17 ausgebildet sein. Figur 2 zeigt ein Detail einer solchen Anordnung in einer Seitenansicht, Figur 3 einen Querschnitt im Bereich eines der Übertragungselemente 15. Der Druck- oder Kraftsensor 13a wird vorzugsweise so im Übertragungsbereich zwischen der Brücke 10 und dem Längsholm 3 angebracht, dass er eine maximale Empfindlichkeit bezüglich Belastungen der Brücke 10 aufweist. Je nach Art des Kraftsensors 13a kann somit z.B. eine Stelle mit relativ grosser elastischer Verformung oder aber eine Stelle mit relativ grossem Auflagedruck vorteilhafter sein. Der Kraftsensor 13a bzw. allgemein der Sensor 13 kann grundsätzlich an unterschiedlichen Stellen des Kraftübertragungsweges zwischen der von der Brücke 10 getragenen Last und der Tragvorrichtung bzw. dem Längsholm 3 angeordnet sein, beispielsweise an der Brücke 10 selbst, an einem der Verbindungsmittel, insbesondere am Übertragungselement 15, am Trägerelement 17 am Längsholm 3 bzw. zwischen zwei benachbarten Teilen im Kraftübertragungsweg.

Der oder die Sensoren 13 bzw. Kraftsensoren 13a sind über eine oder mehrere Verbindungsleitungen 19 mit einer elektronischen Steuerung 21 verbunden, welche die Messgrössen auswertet und Steuergrössen für beispielsweise eine optische und/oder akustische Alarmfunktion erzeugt. Die Steuerung 21 oder Teile dieser Steuerung 21 können auch an der Brücke 10 oder bei einem oder mehreren der Sensoren 13, 13a angeordnet sein. Insbesondere können die Sensoren 13, 13a beispielsweise eine Ansteuer- und/oder Auswerteelektronik wie z.B. einen A/D-Wandler umfassen (nicht dargestellt). Mehrere Sensoren 13, 13a können zur Ansteuerung und/oder Auswertung auch eine gemeinsame Elektronik umfassen (nicht dargestellt). Vorzugsweise ist mindestens ein Teil der Steuerung 21 in einem Gehäuse angeordnet, welches je nach Bedarf an unterschiedlichen Stellen eines Bettes befestigt werden kann, beispielsweise am Kopf- oder Fussende des Bettgestells. Die Steuerung 21 umfasst vorzugsweise eine Anzeige, eine optische und/oder akustische Warneinrichtung, Tasten oder andere Eingabemittel zum Beeinflussen von Gerätefunktionen und eine Schnittstelle, beispielsweise einen Steckeranschluss für eine Ethernet-Verbindung zur Kommunikation mit anderen Geräten (nicht dargestellt).

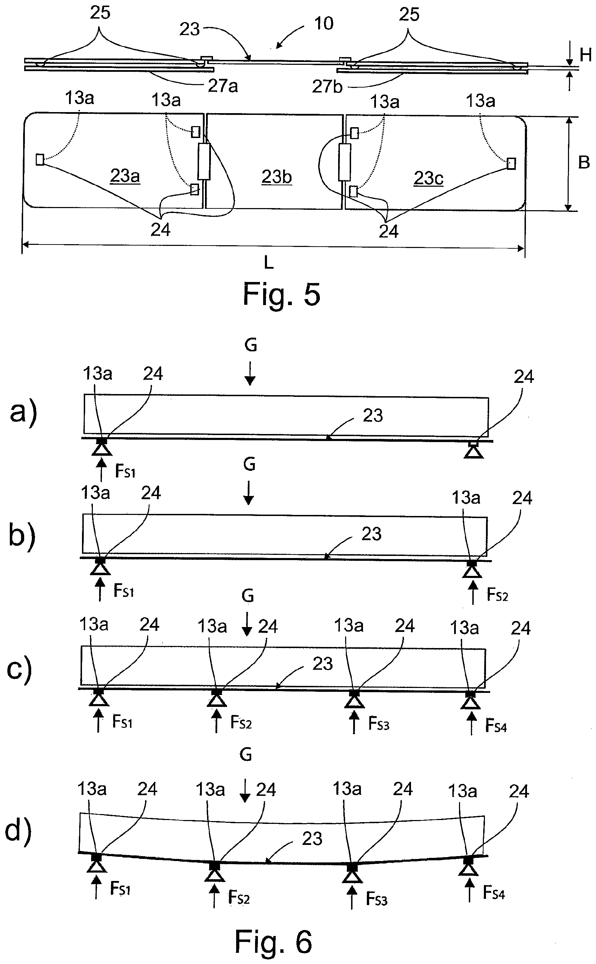

Figur 4 zeigt eine alternative Ausführungsform der Brücke 10 bei einer Liegefläche 1. Diese Brücke 10 ist in Figur 5 in einer Seitenansicht und in Aufsicht dargestellt. Sie umfasst eine näherungsweise rechteckige längliche, vorzugsweise aus Metall gefertigte Platte 23 mit einer Länge L, die etwa der Breite des Bettes entspricht und einer Breite B, die erfindungsgemäss im Bereich von 5 cm bis 40 cm liegt. Die Breite B der Platte 23 kann beispielsweise so bemessen sein, dass sie der Breite des Mittelteils 9 der Liegefläche 1 entspricht. Bei alternativen Ausführungsformen kann die Breite B der Platte 23 beispielsweise auch der Breite einer Bettlatte eines Lattenrosts oder der Breite von zwei oder mehreren Bettlatten einschliesslich des Zwischenraums zwischen diesen Bettlatten entsprechen. Die Platte 23 kann z.B. einstückig oder mehrstückig aus mehreren Abschnitten 23a, 23b, 23c gefertigt sein. Die benachbarten Abschnitte 23a, 23b, 23c sind miteinander verbunden. Zum Herstellen dieser Verbindungen können unterschiedliche Fügetechniken wie z.B. Schweissen, Kleben oder Nieten eingesetzt werden.

Insbesondere können benachbarte Abschnitte 23a, 23b, 23c mittels angeschweisster Stahlbänder gelenkig miteinander verbunden sein. Durch unterschiedliche Dicken und/oder Materialien und/oder Überlappungsbereiche der Abschnitte 23a, 23b, 23c können mechanische Eigenschaften der Brücke 10 wie beispielsweise deren Biegesteifigkeit entsprechend den jeweiligen Anforderungen angepasst werden. Zumindest im Bereich der beiden schmalen Plattenenden können vorzugsweise mittels eines Lasers T-förmige, sprungbrettartige Strukturen in die Platte 23 eingeschnitten sein. Diese Strukturen bilden Stützstellen bzw. Stützbereiche 24, an denen die Platte 23 direkt oder indirekt über geeignete Verbindungsmittel am Bettgerüst bzw. an den Längsholmen 3 abgestützt oder getragen werden kann. Bei jedem dieser Stützbereiche 24, die sich bei Belastung geringfügig elastisch verformen können, ist ein DMS als Kraftsensor 13a angeordnet. Die in den Figuren 4 und 5 dargestellte Brücke 10 umfasst zusätzlich zu den Stützbereichen 24 an den beiden Enden vier weitere Stützbereiche 24. Je zwei davon sind etwa am Ende der beiden äusseren Drittel der gesamten Plattenlänge L angeordnet, also nahe bei den jeweils angrenzenden Abschnitten 23a, 23b, 23c der Platte 23. Die drei linken Stützbereiche 24 sind je über einen Abstandshalter 25 mit einer ersten Kontaktplatte 27a verbunden oder auf dieser ersten Kontaktplatte 27a abgestützt. Diese erste Kontaktplatte 27a ist vorzugsweise in einem geringen Abstand H, der beispielsweise im Millimeterbereich liegen kann, parallel zur Platte 23 ausgerichtet. In analoger Weise sind auch die drei rechten Stützbereiche 24 mit einer zweiten Kontaktplatte 27b verbunden bzw. auf dieser zweiten Kontaktplatte 27b abgestützt. Die Kontaktplatten 27a, 27b haben die Wirkung von Verbindungsmitteln, welche zum Abstützen der Platte 23 bzw. der Brücke 10 an der Tragvorrichtung oder an einer mit der Tragvorrichtung verbundenen Querverbindung bzw. an Bettlatten genutzt werden können. Alternativ oder zusätzlich zu den Stützstellen 24 der Platte 23 können auch die Abstandhalter 25 federnde Strukturen umfassen und mit DMS als Kraftsensoren 13a ausgebildet sein. Beim in Figur 4 dargestellten Beispiel liegt die Brücke 10 auf dem als starre Verbindungsplatte zwischen den Längsholmen 3 ausgebildeten Mittelteil 9 der Liegefläche 1 auf. Da die Brücke einfach auf die Verbindungsplatte aufgelegt werden kann, ist für deren Installation keinerlei Werkzeug erforderlich. Aufgrund ihrer geringen Bauhöhe, die in der Regel weniger als 3 cm, vorzugswiese etwa 1cm bis 1.5cm beträgt, stört die Brücke 10 in keiner Weise. Da die Matratze nun auf der Brücke 10 aufliegt, kann diese Brücke 10 als integraler Bestandteil der Liegefläche 1 oder als auf der Liegefläche 1 aufliegend betrachtet werden. Die Brücke 10 gemäss Figur 5 kann auch einfach auf zwei benachbarte Latten eines Lattenrosts aufgelegt werden, die ohne Belastung eine konvexe oder konkave Wölbung haben. Aufgrund der gelenkigen Verbindung der Abschnitte 23a, 23b, 23c passt sich die Platte 23 so an die Kontur der Latten an, dass die Kontaktplatten 27a, 27b der beiden äusseren Abschnitte 23a, 23c auf den Latten aufliegen, die Platte 23 selbst hingegen die Latten in keinem der Abschnitte 23a, 23b, 23c berührt. Je nach Betrachtungsweise kann die Brücke 10 nur die Platte 23 mit den Stützbereichen 24 umfassen oder alternativ zusätzlich auch die Abstandhalter 25 und die Kontaktplatten 27a, 27b. Im ersten Fall gehören die Abstandhalter 25 und die Kontaktplatten 27a, 27b zu den Verbindungsmitteln, mit denen die Brücke 10 an den Längsholmen 3 der Tragvorrichtung abstützbar ist.

Figur 6 zeigt zum besseren Verständnis unterschiedliche Anordnungen von Platten 23 mit zwei oder mehreren Stützbereichen 24, und mit einem oder mehreren bei solchen Stützbereichen angeordneten Kraftsensoren 13a. Bei der Anordnung a) ist die Platte 23 im Bereich ihrer schmalseitigen Enden an zwei Stützbereichen 24 abgestützt, umfasst aber nur beim linken Stützbereich 24 einen Sensor. Das auf der Platte 23 lastende Gewicht G ist bei beiden Stützbereichen 24 abgestützt, wobei die auf den linken Stützbereich 24 lastende Kraft mit F_{S1} bezeichnet ist. Bei der Anordnung b) ist zusätzlich beim rechten Stützbereich 24 ein Kraftsensor 13a angeordnet, der die zugehörige Stützkraft F_{S2} erfasst. Bei den Anordnungen c) und d) umfasst die Platte 23 drei gelenkig miteinander verbundene Abschnitte 23a, 23b, 23c und vier verteilt über die Länge der Platte 23 angeordnete Stützbereiche 24. Bei jedem dieser Stützbereiche ist ein Kraftsensor 13a angeordnet, der die jeweils zugehörige Stützkraft F_{S1}, F_{S2}, F_{S3}, F_{S4} aufnimmt. Bei der Anordnung c) liegen die Stützbereiche 24 in einer Ebene. Bei der Anordnung d) hingegen liegt die Platte 23 auf einer unebenen Unterlage, beispielsweise auf flexiblen Bettlatten auf. Die Lage der einzelnen Stützbereiche 24 der Platte 23 passen sich an die Form dieser Unterlage an, sodass die Stützbereiche 24 nicht mehr in einer gemeinsamen Ebene liegen.

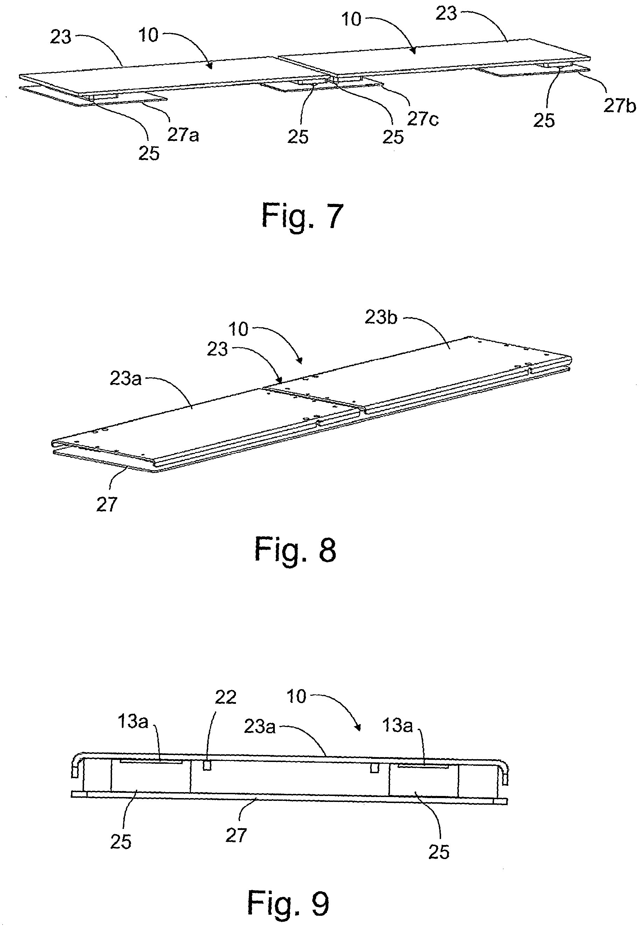

Figur 7 zeigt eine Anordnung zweier Brücken 10, bei denen die benachbarten Enden der Platten 23 über eine mittlere Kontaktplatte 27c indirekt am Bettgestell abgestützt sind (nicht dargestellt). Die beiden äusseren Kontaktplatten 27a, 27b stützen die beiden Platten 23 analog zur Ausführungsform gemäss Figur 5 direkt oder indirekt an den Längsholmen 3 bzw. an der Tragvorrichtung ab.

Figur 8 zeigt eine besonders vorteilhafte Ausgestaltung einer Brücke 10, wobei die Metallplatte zwei Abschnitte 23a, 23b umfasst, bei denen im Bereich der beiden Längskanten je ein schmaler Streifen von etwa 5 bis 15mm Breite um 90° umgeformt bzw. abgekantet ist. Das im Querschnitt U-förmige Profil erhöht die Biegesteifigkeit dieser Plattenabschnitte 23a, 23b. Unter den Plattenabschnitten 23a, 23b erstreckt sich eine Auflage-bzw. Kontaktplatte 27 über die gesamte Länge der Brücke.

Die Kontaktplatte 27 ist formstabil, weist aber eine wesentlich höhere Biegeelastizität auf als die Abschnitte 23a, 23b der oberen Platte 23.

Figur 9 zeigt schematisch die Anordnung aus Figur 8 in einer Seitenansicht. Die Abschnitte 23a, 23b werden mittels Stützkörpern bzw. Abstandhaltern 25 auf der Kontaktplatte 27 abgestützt. Die Kraftsensoren 13a sind zwischen den Abstandhaltern 25 und dem jeweiligen oberen Plattenabschnitt 23a, 23b angeordnet. Die Abstandhalter 25 sind mit den jeweiligen oberen Plattenabschnitten 23a, 23b verbunden und liegen vorzugsweise nur lose auf der Kontaktplatte 27 auf. Diese Anordnung ist in einer (nicht dargestellten) Schutzhülle verpackt. Bei lose angeordneten Platten 23, Plattenabschnitten 23a, 23b, 23c und/oder Kontaktplatten 27 hält die Schutzhülle diese Elemente auch zusammen. Der Innenraum der Schutzhülle ist in Form und Grösse an die Abmessungen der Brücke 10 angepasst. Insbesondere kann die Schutzhülle eine mit einem Klett- oder Reissverschluss verschliessbare Öffnung und/oder Innentaschen zum Aufnehmen und Positionieren von Plattenabschnitten 23a, 23b, 23c umfassen (nicht dargestellt). Nebst den umgeformten Längskanten umfassen die Plattenabschnitte 23a, 23b weitere die Biegesteifigkeit erhöhende Elemente in Gestalt von Längsrippen 22, die an den unteren Oberflächen der Plattenabschnitte 23a, 23b angeklebt sind. Selbst bei Abstandhaltern mit geringen Höhen können Brücken 10 hergestellt werden, deren biegesteife Plattenabschnitte 23a, 23b auch beim Auflegen auf gewölbte Liegeflächen die unteren Kontaktplatten 27 nicht berühren.

## Patentansprüche

1. Messvorrichtung zum Erfassen von Lageänderungen von Personen in einem Bett, wobei dieses Bett eine von einer Tragvorrichtung getragene Liegefläche (1) und eine darauf liegenden Matratze umfasst, wobei mindestens eine längliche Brücke (10), die zum Stützen der Matratze in einem mittleren Bereich zwischen einem Kopfbereich und einem Fussbereich des Bettes anordnenbar ist, eine geringe Breite im Bereich von 5cm bis 40cm aufweist und zum Übertragen der von der Brücke (10) gestützten Last an die Tragvorrichtung ausgebildet ist, wobei diese Brücke (10) mindestens eine längliche Platte (23) mit mindestens einem Abschnitt (23a, 23b, 23c) umfasst, der abgekantete Längskanten und/oder in Längsrichtung der Platte (23) verlaufende Verstärkungsrippen als Strukturen oder Elemente zur Erhöhung der Biegesteifigkeit aufweist, und wobei im Kraftübertragungsweg der von der Brücke (10) gestützten Last mindestens ein Sensor (13, 13a) zum Erfassen einer Messgrösse angeordnet ist, die durch die Lastverteilung der von der Brücke (10) gestützten Last beeinflussbar ist, wobei die Messvorrichtung eine Steuerung (21) umfasst, mit welcher der oder die Sensoren (13, 13a) verbunden sind, wobei diese Steuerung (21) ganz oder teilweise in einem unabhängig von der Brücke (10) platzierbaren Gehäuse oder ganz oder teilweise an der Brücke (10) angeordnet ist, und wobei diese Steuerung (21) Mittel zur Erfassung und Auswertung der Messgrössen der Sensoren (13, 13a) umfasst, **dadurch gekennzeichnet, dass** die Mittel zur Erfassung und Auswertung der Messgrössen der Sensoren ausgebildet sind als Mittel zur Erfassung der Schwerpunktlage oder von Änderungen der Schwerpunktlage der auf der Brücke (10) lastenden Gewichtsverteilung sowie Mittel zum optischen und/oder akustischen und/oder elektrischen Anzeigen einer Bewegungsaktivität zur Einschätzung des Dekubitus-Risikos umfasst.

2. Messvorrichtung nach Anspruch 1, **dadurch gekennzeichnet, dass** der Sensor (13, 13a) oder mindestens einer der Sensoren (13, 13a) ein Kraftsensor (13a), insbesondere ein mit Druck und/oder Zug beaufschlagbarer resistiver Kraftsensor (13a) oder Dehnmessstreifen ist.

3. Messvorrichtung nach einem der Ansprüche 1 bis 2, **dadurch gekennzeichnet, dass** die Brücke (10) mindestens zwei voneinander beabstandete Stützbereiche (24) umfasst, wobei diese Stützbereiche (24)
a) direkt von der Tragvorrichtung stützbar sind, und der oder die Sensoren (13, 13a) bei dem oder den Stützbereichen (24) an der Brücke (10) oder an der Tragvorrichtung oder zwischen der Brücke (10) und der Tragvorrichtung angeordnet sind, oder
b) indirekt durch Verbindungsmittel von der Tragvorrichtung stützbar sind, und der oder die Sensoren (13, 13a) bei dem oder den Stützbereichen (24) an der Brücke (10) oder an den Verbindungsmitteln oder zwischen der Brücke (10) und den Verbindungsmitteln oder zwischen den Verbindungsmitteln und der Tragvorrichtung oder an der Tragvorrichtung angeordnet sind.

4. Messvorrichtung nach Anspruch 3, soweit sich dieser auf Anspruch 2 zurückbezieht, **dadurch gekennzeichnet, dass** mindestens einer der Stützbereiche (24) der Platte (23) mittels eines Abstandhalters (25) direkt oder indirekt an der Tragvorrichtung abstützbar ist, dass dieser Stützbereich (24) oder der Abstandhalter (25) eine federnde Struktur umfasst, und dass der mindestens eine Sensor (13, 13a) an der Platte (23) oder am Abstandhalter (25) bei dieser federnden Struktur angeordnet ist.

5. Messvorrichtung nach Anspruch 3, wobei die Platte (23) zwei oder mehrere biegesteife Abschnitte (23a, 23b, 23c) umfasst, **dadurch gekennzeichnet, dass** jeweils benachbarte Abschnitte (23a, 23b, 23c) gelenkig oder flexibel miteinander verbunden oder lose nebeneinander angeordnet sind.

6. Messvorrichtung nach Anspruch 5, **dadurch gekennzeichnet, dass** zumindest an einem der beiden äussersten Abschnitte (23a, 23b, 23c) der Platte (23) mindestens zwei zueinander beabstandete Stützbereiche (24) ausgebildet sind, dass die Stützbereiche (24) dieses Abschnittes (23a, 23b, 23c) mittels Abstandhaltern (25) auf einer gemeinsamen, dem jeweiligen Abschnitt (23a, 23b, 23c) zugeordneten Kontaktplatte (27a, 27b) abgestützt sind, und dass die Stützbereiche (24) oder die Abstandhalter (25) federnde Strukturen mit Sensoren (13, 13a) umfassen.

7. Messvorrichtung nach Anspruch 3, wobei die Stützbereiche (24) indirekt über Verbindungsmittel von der Tragvorrichtung stützbar sind, **dadurch gekennzeichnet, dass** mindestens eines der Verbindungsmittel ein an der Trägervorrichtung befestigbares Trägerelement (17) ist, und dass mindestens ein Sensor (13, 13a) an diesem Trägerelement (17) angeordnet ist.

8. Messvorrichtung nach einem der Ansprüche 1 bis 7, **dadurch gekennzeichnet, dass** die Bauhöhe der Brücke (10) kleiner ist als 3cm.

9. Messvorrichtung nach Anspruch 8, **dadurch gekennzeichnet, dass** die Steuerung (21) eine Kommunikationsschnittstelle umfasst.

10. Bett mit einer Messvorrichtung nach einem der Ansprüche 1 bis 9, umfassend eine Liegefläche (1) mit einem Rückenteil (5), einem Mittelteil (9) und einem Fussteil (7), **dadurch gekennzeichnet, dass** die Brücke (10) am Mittelteil (9) der Liegefläche (1) ausgebildet oder angeordnet und quer zur Längsrichtung der Liegefläche (1) ausgerichtet ist.

11. Verfahren zum Verarbeiten von Messgrössen bei einem Bett nach Anspruch 10, **dadurch gekennzeichnet, dass** anhand der Messgrössen des oder der Sensoren (13, 13a) die relative Lage des Schwerpunktes des im mittleren Bereich des Bettes auf der Brücke (10) lastenden Gewichts quer zur Längsrichtung der Liegefläche (1) ermittelt wird, dass die Werte der Schwerpunktlage und/oder die Werte der Messgrössen des oder der Sensoren (13, 13a) periodisch gespeichert werden, dass Änderungen der Schwerpunktlage überwacht werden, dass eine ungenügende Bewegungsaktivität durch Vergleich mit gespeicherten Kriterien ermittelt wird, und dass bei ungenügender Bewegungsaktivität ein Alarm ausgelöst wird.

## Claims

1. A measuring device for detecting positional changes of persons in a bed,
wherein said bed comprises a lying surface (1) carried by a carrying device and a mattress positioned thereon,
wherein at least one longitudinal bridge (10), which is able to be arranged in a center region between a head region and a foot region of the bed in order to support the mattress, has a narrow width in the range of 5 cm to 40 cm and is designed to transmit the load supported by the bridge (10) to the carrying device,
wherein said bridge (10) comprises at least one longitudinal panel (23) having at least one portion (23a, 23b, 23c) that has bent-over longitudinal edges and/or reinforcing ribs extending in the longitudinal direction of the panel (23) as structures or elements for increasing the flexural rigidity, and
wherein, in the force transmission path of the load supported by the bridge (10), at least one sensor (13, 13a) for recording a measured variable is arranged, said measured variable being capable of being influenced by the load distribution of the load supported by the bridge (10),
wherein the measuring device comprises a controller (21), to which the sensor(s) (13, 13a) is/are connected,
wherein said controller (21) is arranged entirely or in part in a housing that is able to be placed independently of the bridge (10) or entirely or in part on the bridge (10), and wherein said controller (21) comprises means for detecting and analyzing the measured variables from the sensors (13, 13a),
**characterized in that** the means for detecting and analyzing the measured variables from the sensors are designed as means for detecting the center of gravity or changes in the center of gravity of the weight distribution bearing on the bridge (10) and means for optically and/or acoustically and/or electrically displaying movement activity for assessing the risk of decubitus.

2. The measuring device according to Claim 1, **characterized in that** the sensor (13, 13a) or at least one of the sensors (13, 13a) is a force sensor (13a), in particular a resistive force sensor (13a) to which pressure and/or tension can be applied or a strain gage.

3. The measuring device according to any one of Claims 1 to 2, **characterized in that** the bridge (10) comprises at least two supporting regions (24) spaced apart from one another, wherein said supporting regions (24)
a) are able to be directly supported by the carrying device, the sensor(s) (13, 13a) being arranged on the bridge (10) or on the carrying device or between the bridge (10) and the carrying device, proximal to the supporting region(s) (24), or
b) is able to be indirectly supported by connection means of the carrying device, the sensor(s) (13, 13a) being arranged on the bridge (10) or on connection means or between the bridge (10) and the connection means or between the connection means and the carrying device or on the carrying device, proximal to the supporting region(s) (24).

4. The measuring device according to Claim 3, insofar as said claim refers to Claim 2, **characterized in that** at least one of the supporting regions (24) of the panel (23) is able to be directly or indirectly supported on the carrying device by means of a spacer (25), **in that** said supporting region (24) or the spacer (25) comprises a resilient structure, and **in that** the at least one sensor (13, 13a) is arranged on the panel (23) or on the spacer (25) proximal to said resilient structure.

5. The measuring device according to Claim 3, wherein the panel (23) comprises two or more flexurally rigid portions (23a, 23b, 23c), **characterized in that** adjacent portions (23a, 23b, 23c) are hingedly or flexibly interconnected or arranged loosely one next to the other.

6. The measuring device according to Claim 5, **characterized in that**, on at least one of the two outermost portions (23a, 23b, 23c) of the panel (23), at least two spaces apart supporting regions (24) are formed, **in that** the supporting regions (24) of said portion (23a, 23b, 23c) are supported on a common contact panel (27a, 27b) associated with the particular portion (23a, 23b, 23c) by means of spacers (25), and **in that** the supporting regions (24) or the spacers (25) comprise resilient structures having sensors (13, 13a).

7. The measuring device according to Claim 3, wherein the supporting regions (24) are able to be indirectly supported by connection means of the supporting device, **characterized in that** at least one of the connection means is a carrier element (17) that is able to be fastened to the carrier device, and **in that** at least one sensor (13, 13a) is arranged on said carrier element (17).

8. The measuring device according to any one of Claims 1 to 7, **characterized in that** the overall height of the bridge (10) is less than 3 cm.

9. The measuring device according to Claim 8, **characterized in that** the controller (21) comprises a communication interface.

10. A bed having a measuring device according to any one of Claims 1 to 9, comprising a lying surface (1) having a rear part (5), a center part (9) and a foot part (7), **characterized in that** the bridge (10) is formed or arranged on the center part (9) of the lying surface (1) and is arranged transversely to the longitudinal direction of the lying surface (1).

11. A method for processing measured variables in a bed according to Claim 10, **characterized in that**, on the basis of the measured variables from the sensor(s) (13, 13a), the relative position of the center of gravity of the weight bearing on the bridge (10) in the center region of the bed, transverse to the longitudinal direction of the lying surface (1), is determined, **in that** the values for the center of gravity and/or the values for the measured variables from the sensor(s) (13, 13a) are/is periodically stored, **in that** changes in the center of gravity are monitored, **in that** insufficient movement activity is ascertained by comparison with stored criteria, and **in that** an alarm is triggered in the event of insufficient movement activity.

## Revendications

1. Dispositif de mesure pour détecter des modifications de position de personnes dans un lit, ce lit comprenant une surface de couchage (1) portée par un dispositif porteur et une matelas situé sur celle-ci,
au moins un pont allongé (10), qui peut être agencé pour le soutien du matelas dans une zone centrale entre la zone de tête et une zone de pied du lit, présentant une faible largeur dans la plage allant de 5 cm à 40 cm et étant configuré pour le transfert de la charge soutenue par le pont (10) au dispositif porteur,
ce pont (10) comprenant au moins une plaque allongée (23) munie d'au moins une section (23a, 23b, 23c), qui comprend des bords longitudinaux biseautés et/ou des nervures de renforcement s'étendant dans la direction longitudinale de la plaque (23) en tant que structures ou éléments pour augmenter la rigidité en flexion, et
au moins un capteur (13, 13a) pour la détection d'une grandeur de mesure étant agencé dans la trajectoire de transfert de force de la charge soutenue par le pont (10), qui peut être influencé par la répartition de charge de la charge soutenue par le pont (10), le dispositif de mesure comprenant une commande (21), avec laquelle le ou les capteurs (13, 13a) (21) sont reliés, cette commande (21) étant agencée en totalité ou en partie dans un boîtier pouvant être placé indépendamment du pont (10) ou en totalité ou en partie sur le pont (10), et cette commande (21) comprenant des moyens pour la détection et l'évaluation des grandeurs de mesure des capteurs (13, 13a), **caractérisé en ce que** les moyens pour la détection et l'évaluation des grandeurs de mesure des capteurs sont configurés en tant que moyens pour la détection de la position du centre de gravité ou de modifications de la position du centre de gravité de la distribution de poids chargée sur le pont (10), ainsi que moyens pour l'indication optique et/ou acoustique et/ou électrique d'une activité de mouvement pour l'estimation du risque de décubitus.

2. Dispositif de mesure selon la revendication 1, **caractérisé en ce que** le capteur (13, 13a) ou au moins un des capteurs (13, 13a) est un capteur de force (13a), notamment un capteur de force (13a) résistif pouvant être sollicité en pression et/ou traction ou une jauge de contrainte.

3. Dispositif de mesure selon l'une quelconque des revendications 1 à 2, **caractérisé en ce que** le pont (10) comprend au moins deux zones de soutien (24) espacées l'une de l'autre, ces zones de soutien (24)
a) pouvant être soutenues directement par le dispositif porteur, et le ou les capteurs (13, 13a) étant agencés dans la ou les zones de soutien (24) sur le pont (10) ou sur le dispositif porteur ou entre le pont (10) et le dispositif porteur, ou
b) pouvant être soutenues indirectement par le biais de moyens de liaison par le dispositif porteur, et le ou les capteurs (13, 13a) étant agencés dans la ou les zones de soutien (24) sur le pont (10) ou sur les moyens de liaison ou entre le pont (10) et les moyens de liaison ou entre les moyens de liaison et le dispositif porteur ou sur le dispositif porteur.

4. Dispositif de mesure selon la revendication 3, dans la mesure où celle-ci se rapporte à la revendication 2, **caractérisé en ce qu'**au moins une des zones de soutien (24) de la plaque (23) peut être soutenue directement ou indirectement sur le dispositif porteur au moyen d'un espaceur (25), **en ce que** cette zone de soutien (24) ou l'espaceur (25) comprend une structure élastique, et **en ce que** l'au moins un capteur (13, 13a) est agencé sur la plaque (23) ou sur l'espaceur (25) dans cette structure élastique.

5. Dispositif de mesure selon la revendication 3, dans lequel la plaque (23) comprend deux sections rigides en flexion (23a, 23b, 23c) ou plus, **caractérisé en ce que** des sections voisines (23a, 23b, 23c) sont à chaque fois reliées les unes aux autres de manière souple ou flexible ou agencées de manière lâche les unes à côté des autres.

6. Dispositif de mesure selon la revendication 5, **caractérisé en ce qu'**au moins deux zones de soutien espacées l'une de l'autre (24) sont formées au moins sur une des deux sections les plus extérieures (23a, 23b, 23c) de la plaque (23), **en ce que** les zones de soutien (24) de cette section (23a, 23b, 23c) sont soutenues au moyen d'espaceurs (25) sur une plaque de contact commune (27a, 27b), attribuée à la section respective (23a, 23b, 23c), et **en ce que** les zones de soutien (24) ou les espaceurs (25) comprennent des structures élastiques munies de capteurs (13, 13a).

7. Dispositif de mesure selon la revendication 3, dans lequel les zones de soutien (24) peuvent être soutenues indirectement par l'intermédiaire de moyens de liaison par le dispositif porteur, **caractérisé en ce qu'**au moins un des moyens de liaison est un élément support (17) pouvant être fixé au dispositif support, et **en ce qu'**au moins un capteur (13, 13a) est agencé sur cet élément support (17).

8. Dispositif de mesure selon l'une quelconque des revendications 1 à 7, **caractérisé en ce que** la hauteur de construction du pont (10) est inférieure à 3 cm.

9. Dispositif de mesure selon la revendication 8, **caractérisé en ce que** la commande (21) comprend une interface de communication.

10. Lit muni d'un dispositif de mesure selon l'une quelconque des revendications 1 à 9, comprenant une surface de couchage (1) munie d'une partie de dos (5), d'une partie centrale (9) et d'une partie de pied (7), **caractérisé en ce que** le pont (10) est formé ou agencé sur la partie centrale (9) de la surface de couchage (1) et est dirigé perpendiculairement à la direction longitudinale de la surface de couchage (1).

11. Procédé pour le traitement de grandeurs de mesure dans un lit selon la revendication 10, **caractérisé en ce qu'**à partir des grandeurs de mesure du ou des capteurs (13, 13a), la position relative du centre de gravité du poids chargé dans la zone centrale du lit sur le pont (10) perpendiculairement à la direction longitudinale de la surface de couchage (1) est déterminé, **en ce que** les valeurs de la position du centre de gravité et/ou les valeurs des grandeurs de mesure du ou des capteurs (13, 13a) sont mémorisées périodiquement, **en ce que** des modifications de la position du centre de gravité sont surveillées, **en ce qu'**une activité de mouvement insuffisante est déterminée par comparaison avec des critères mémorisés, et **en ce qu'**en cas d'activité de mouvement insuffisante, une alarme est déclenchée.
